# FASCICULE DE BREVET EUROPEEN

(11) **EP 2 570 043 B1**
(45) Date de publication et mention de la délivrance du brevet: **06.07.2016**
(21) Numéro de dépôt: 12006620.4
(22) Date de dépôt: 10.11.2010
(51) Int. Cl.: B29C 35/08, B29K 23/00, B29C 47/30, B29L 9/00, B29C 59/14, B29C 61/02, B29C 35/04

(54) **Installation de fabrication d'une partie femelle à boucles à film et filaments ancrés par thermorétraction**
Anlage zur Herstellung eines Schlaufenteils eines Klettverschusses mit Folie und durch Thermoschrumpfung verankerten Fasern
Installation for producing a female loop portion having film and filaments anchored by heat-shrinking

(30) Priorité: 20.11.2009 FR 0905588
(43) Date de publication de la demande: 20.03.2013
(62) Demande divisionnaire de: 10787513.0
(73) Titulaire: Aplix, 75008 Paris (FR)
(72) Inventeur: Ducauchuis, Jean-Pierre, 44100 Nantes (FR); Mahe, Anthony, 44450 St Julien de Concelles (FR)
(74) Mandataire: Eidelsberg, Olivier Nathan

(56) Documents cités:
- EP-A1- 0 289 198
- FR-A1- 2 870 436
- US-A- 5 611 791

## Description

La présente invention se rapporte à une partie femelle à boucles pour un dispositif de fermeture autoagrippant à crochets et boucles, notamment en vue d'une utilisation pour la fermeture de couche-culottes, cette partie femelle étant notamment destinée à être fixée à la ceinture de la couche-culotte centralement dans la zone du bas ventre du porteur de la couche-culotte, zone communément appelée bande confort ou Landing Zone (en anglais).

La présente invention se rapporte également à un procédé de fabrication d'une partie femelle à boucles de ce genre, ainsi qu'à une installation pour. la mise en oeuvre d'un procédé de ce genre.

On connaît déjà dans l'art antérieur une partie femelle comportant un film en matière thermoplastique sur lequel des filaments individuels ont été ancrés suivant des sections d'ancrage pour former des boucles avec les parties des filaments restants non ancrées. On pourra notamment se référer au brevet français publié sous le numéro 2 870 436 qui est au nom de la demanderesse.

Une installation destinée à fabriquer un élément femelle à boucle selon le préambule de la revendication 1 est décrit dans le document US-5.611.791-A.

On souhaiterait obtenir une partie femelle à boucles du genre de l'art antérieur décrit ci-dessus qui ait des filaments qui soient aussi bien fixés au film en matière thermoplastique que dans l'art antérieur et qui en outre résistent mieux à la traction, notamment lorsque les boucles coopèrent avec des crochets.

Suivant un premier aspect de l'invention, la présente invention se rapporte à une installation destinée à fabriquer un élément femelle à boucle telle que définie à la revendication 1, des perfectionnements étant définis aux sous revendications.

En prévoyant ainsi de préfixer les filaments tout en en conservant leur intégrité par rapport au film de sorte que les matières ne soient pas intimement liées, notamment dans une mesure telle qu'ils sont fixés au film mais peuvent en être détachés facilement en conservant sinon leur forme initiale(en général la section a été déformée) du moins leur intégrité, puis de réaliser une thermo rétraction à une température qui est supérieure aux températures de ramollissement des filaments et du film mais inférieure aux points de fusion de l'un et de l'autre, on obtient une rétraction de la matière du film et une liaison particulièrement résistante dans les zones de préfixation des filaments à la matière du film et ce, sans que soient dégradés ni les bases des boucles ni le film en matière thermoplastique.

En effet, dans l'art antérieur, les procédés pour ancrer les filaments à la matière thermoplastique du film consistaient principalement en une soudure ou un calandrage qui dégradaient les filaments notamment au niveau des pieds des boucles, en ayant tendance à les amincir. En outre ces traitements avaient tendance à dégrader le film au point de le percer, ce qui nécessitait de prévoir des films de grandes épaisseurs. Au contraire, suivant l'invention, on obtient une résistance plus élevée des boucles à la traction. En outre, à la fin du traitement le film ne s'amincit pas, ou pour le moins s'amincit moins que dans l'art antérieur, par rapport à son épaisseur avant le traitement thermique, de sorte qu'on peut prévoir d'utiliser au départ des films plus minces que dans l'art antérieur ou des films aussi minces que dans l'art antérieur sans craindre un percement du film.

Suivant un mode de réalisation préféré, le caractère thermo rétractable du film est obtenu par le fait, avant le dépôt du faisceau ou ensemble de filaments, de l'étirer, notamment par un étirage dit « court ».

De préférence, la préfixation s'effectue par une étape dans laquelle les tronçons de préfixation des filaments sont pressés contre le film tandis que dans le même temps, les tronçons de boucle des filaments ne sont pas pressés contre le film.

En particulier, le pressage s'effectue par un cylindre gravé comportant des zones de surélévation correspondant aux zones destinées à presser les tronçons de préfixation lorsque le film et les filaments sont pris en sandwich entre le cylindre gravé et une surface opposée de support pouvant notamment être constituée d'un cylindre, qui peut être également gravé.

De préférence, le cylindre gravé de pressage est chauffé, notamment à une température supérieure à la température de déformation des polymères employés mais cependant en dessous des Points Vicat A des matières du film et des filaments.

Suivant un deuxième aspect de l'invention, indépendant du premier aspect décrit ci-dessus, et formant une invention en tant que tel mais qui peut être mis en oeuvre en combinaison avec le premier aspect pour le perfectionner, les filaments du faisceau ou ensemble de filaments sont réalisés directement par extrusion, avant dépôt sur le film en matière thermoplastique.

Suivant un troisième aspect de l'invention, indépendant des premier et deuxième aspects, et formant une invention en tant que tel mais qui peut également être mis en oeuvre en combinaison avec l'un quelconque des deux premier et deuxième aspects ou avec les deux à la fois en vue de les perfectionner, les filaments sont réalisés en POY (Pre Oriented Yarn) ou LOY (Low Oriented Yarn).

Le POY est un fil ou filament en matériau classiquement utilisé dans la filature, par exemple thermoplastique tel que du polyester, mais qui lors de sa fabrication n'a pas subi la totalité des étapes d'étirage, comme décrit dans de nombreux brevets américains, par exemple US 4736500, US 4244174 ou US 4415521.

Suivant un quatrième aspect de l'invention, indépendant des autres aspects, et formant une invention en tant que tel mais qui peut également être mis en oeuvre en combinaison avec l'un quelconque ou plusieurs des autres aspects en vue de les perfectionner, les tronçons de préfixation sont décalés mutuellement entre deux filaments successifs dans la direction longitudinale des filaments.

En prévoyant ainsi un décalage des tronçons de préfixation d'un filament au suivant dans la succession de filaments du faisceau de filaments dans la direction longitudinale dans laquelle s'étendent les filaments, on influe sur l'orientation finale des boucles formées par les tronçons de boucles dans le produit final, après le traitement thermique. En effet, en fonction de ce décalage des sections de préfixation, les boucles auront tendance à s'orienter vers la gauche ou vers la droite par rapport à la direction longitudinale dans laquelle s'étendent les filaments.

La présente invention se rapporte également à une partie femelle à boucles pouvant être obtenue par un des procédés suivant l'invention.

Suivant un cinquième aspect de l'invention, indépendant des autres aspects, et formant une invention en tant que tel mais qui peut également être mis en oeuvre en combinaison avec l'un quelconque ou plusieurs des autres aspects en vue de les perfectionner, une partie femelle à boucles pour un autoagrippant à crochets et boucles, comportant un film en au moins une matière thermoplastique et une pluralité de filaments, indépendants les uns des autres et fixés à l'une des faces du film, chaque filament comportant une succession de tronçons de fixation et une succession de tronçons de boucles en alternance avec les tronçons de fixation, les filaments étant fixés au film le long des tronçons de fixation tandis que les tronçons de boucles sont à distance du film pour former des boucles, est caractérisée en ce que l'aire d'une section transversale d'un tronçon de boucles d'un filament est inférieure à l'aire d'une section transversale d'un tronçon de fixation dudit un filament.

De préférence, la plus grande des aires de section transversale d'un tronçon de boucle est inférieure à la plus petite des aires de section transversale d'un tronçon de fixation.

De préférence, un tronçon en forme de bulbe s'étend entre un tronçon de boucle et un tronçon de fixation, le tronçon en forme de bulbe s'évasant du tronçon de boucle vers le tronçon de fixation.

De préférence, l'aire de la section transversale d'un tronçon de boucle est constante, en particulier en étant de forme circulaire.

De préférence, l'aire de la section transversale d'un tronçon de fixation est constante, en particulier en étant de forme oblongue, par exemple elliptique ou ovale.

Suivant un sixième aspect, indépendant des autres aspects, et formant une invention en tant que tel mais qui peut également être mis en oeuvre en combinaison avec l'un quelconque ou plusieurs des autres aspects en vue de les perfectionner, les filaments sont en POY ou en LOY.

Suivant un septième aspect de l'invention, indépendant des autres aspects et formant une invention en tant que tel mais qui peut également être mis en oeuvre en combinaison avec l'un quelconque ou plusieurs des autres aspects en vue de les perfectionner, les tronçons de fixation sont décalés mutuellement dans la direction longitudinale entre deux filaments successifs.

De préférence, les filaments s'étendent parallèlement les uns aux autres.

De préférence, les tronçons de fixation des filaments s'étendent parallèlement à une distance axe à axe l'un de l'autre qui est comprise entre 0,02 mm et 0,5mm, de préférence entre 0,05mm et 0,15mm, et notamment supérieure à 0,1 mm.

De préférence, la longueur sur laquelle s'étend chaque tronçon de fixation est comprise entre 0,1 mm et 1 mm, par exemple et préférentiellement 0,25 mm, tandis que la longueur de chaque tronçon formant les boucles est comprise entre 0,5 mm et 5 mm, par exemple et préférentiellement 1 mm.

Suivant un mode de réalisation préféré de l'invention, les filaments ont un titre compris entre 5 et 15 Dtex, notamment égal à 8 Dtex. Il s'agit en particulier du titre qui correspond au titre des filaments au niveau des tronçons de boucles (notamment hors bulbe), sachant que les tronçons de fixation ont un titre qui est supérieur, car en raison du procédé suivant l'invention ils ont été « gonflés ».

De préférence, vu de dessus, le tronçon de fixation a une plus grande largeur, ou dimension transversale perpendiculairement à la direction longitudinale dans laquelle s'étend le filament, qui est supérieure à la largeur du tronçon de boucle du filament.

Suivant l'invention, on entend par indépendants les uns des autres, des tronçons de boucle de filament qui ne sont pas liés à leurs voisins, (par exemple comme cela serait le cas s'ils en formaient une feuille). Les tronçons de boucles sont reliés entre eux uniquement par le film en matière plastique mais pas entre eux directement, même s'il peut arriver que certains tronçons de boucle viennent en contact avec d'autres. Cependant s'il peut y avoir contact entre eux , il n'y a pas pour autant fixation au niveau de ces contacts et les tronçons de boucles peuvent être éloignés les uns des autres.

Suivant un huitième aspect de l'invention, indépendant des autres aspects et formant une invention en tant que tel mais qui peut également être mis en oeuvre en combinaison avec l'un quelconque ou plusieurs des autres aspects en vue de les perfectionner, un procédé de fabrication d'une partie femelle d'auto agrippant, comportant un film en une ou plusieurs matière(s) thermoplastique(s) et des filaments en une ou plusieurs matière(s) thermoplastique(s) fixés sur une des faces du film pour former des boucles, est caractérisé en ce qu'il comporte les étapes qui consistent :
- à prendre un film thermo rétractable constitué d'une ou de plusieurs matière(s) thermoplastique(s),
- à déposer sur le film un faisceau ou ensemble de filaments constitués d'une ou de plusieurs matière(s) thermoplastique (s) et non liés les uns aux autres, la ou les matière(s) du film et la ou les matière(s) des filaments étant choisies de sorte que le film soit plus thermo rétractable que les filaments et notamment soit thermo rétractable tandis que les filaments ne le sont sensiblement pas,
- à réaliser une préfixation le long de chaque filament sur une série de sections de préfixation alternant avec une série de sections libres dans lesquelles le filament n'est pas fixé au film thermoplastique, cette préfixation étant réalisée de manière à ce que les matières des filaments et du film ne soient pas intimement liées ; et
- à soumettre l'ensemble film et filaments préfixés à un traitement thermique entraînant la rétraction du film et le ramollissement des filaments pour obtenir la partie femelle à boucles, le traitement thermique étant réalisé par chauffage à une température qui est sensiblement égale ou supérieure au Point Vicat A de la matière ou d'au moins l'une des matières constituant les filaments et au Point Vicat A de la matière ou d'au moins l'une des matières constituant le film et qui est inférieure à au moins un des points de fusion des matières des filaments et du film.

A titre d'illustration uniquement, on décrit maintenant des modes de réalisation des différents aspects de l'invention en se reportant aux dessins dans lesquels ;
La figure 1 représente, en coupe longitudinale, l'état d'une partie d'un élément à boucles lors de sa fabrication, après l'étape de préfixation ;
La figure 2 représente, en coupe longitudinale, la même partie de l'élément de la figure 1 tel qu'obtenue à la fin du procédé de fabrication ;
La figure 3 représente schématiquement en coupe transversale la partie de l'élément de la figure 17 suivant la ligne AA.;
La figure 4 est une représentation schématique d'un autre mode de réalisation d'un élément suivant l'invention ;
La figure 5 est une vue identique aux figures 3 ou 4 d'encore un autre mode de réalisation d'un élément suivant l'invention ;
La figure 6 est un graphe représentant le retrait du film en fonction de la température du traitement thermique, le graphe permettant de définir la plage optimale (en hachuré) pour la température du traitement thermique suivant l'invention.
La figure 7 est une vue en perspective et en coupe à plus grande échelle de l'élément de la figure 2 ;
Les figures 8A à 8C sont des vues de dessus de différentes configurations de disposition des boucles ;
Les figures 9A à 9C sont des vues de côté des dispositions des figures 8A à 8C ;
La figure 10 est un schéma explicatif d'un premier procédé suivant l'invention pour orienter les boucles ;
La figure 11 est un schéma explicatif d'un deuxième procédé suivant l'invention pour orienter les boucles ;
Les figures 12 à 14 représentent schématiquement un élément à boucles lors de différentes étapes de fabrication, la figure 12 représentant le film seul avant que soient extrudés des filaments sur le film, la figure 13 représentant l'état de l'élément femelle une fois que les filaments ont été positionnés sur le film et préfixés, et la figure 14 représentant l'élément à boucles obtenu à la fin du procédé ;
La figure 15 représente une installation pour fabriquer un élément à boucle suivant l'invention ;
La figure 16 est une vue de côté d'une partie de l'installation de la figure 15 au niveau où s'effectue la préfixation des filaments au film ;
La figure 17 est une vue de dessus d'une partie d'un élément à boucle une fois la préfixation réalisée et avant l'étape de traitement thermique ; et
La figure 18 représente vu de dessus une bande confort suivant l'invention comportant des boucles agencées suivant un motif particulièrement avantageux.

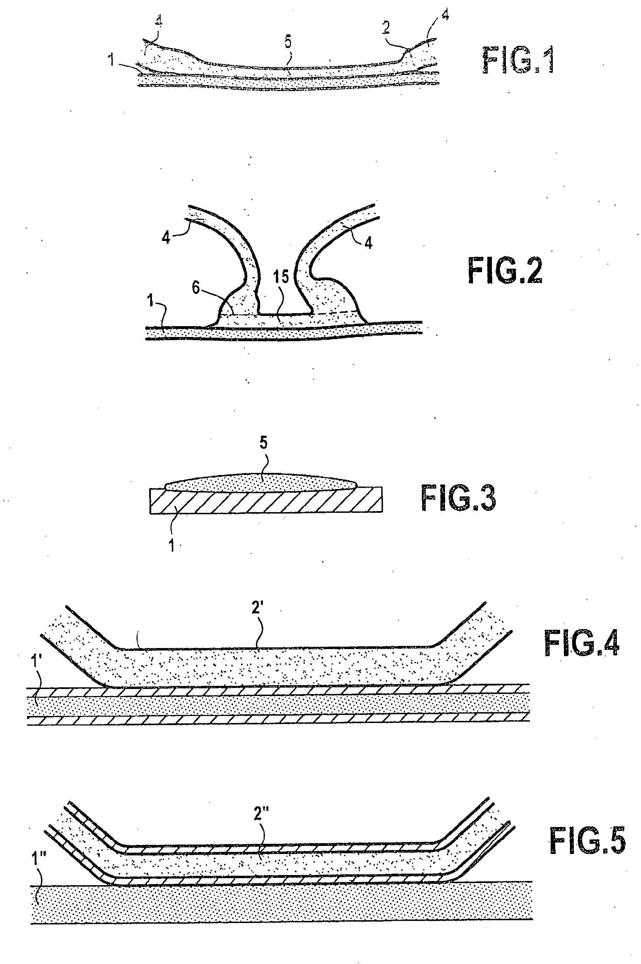

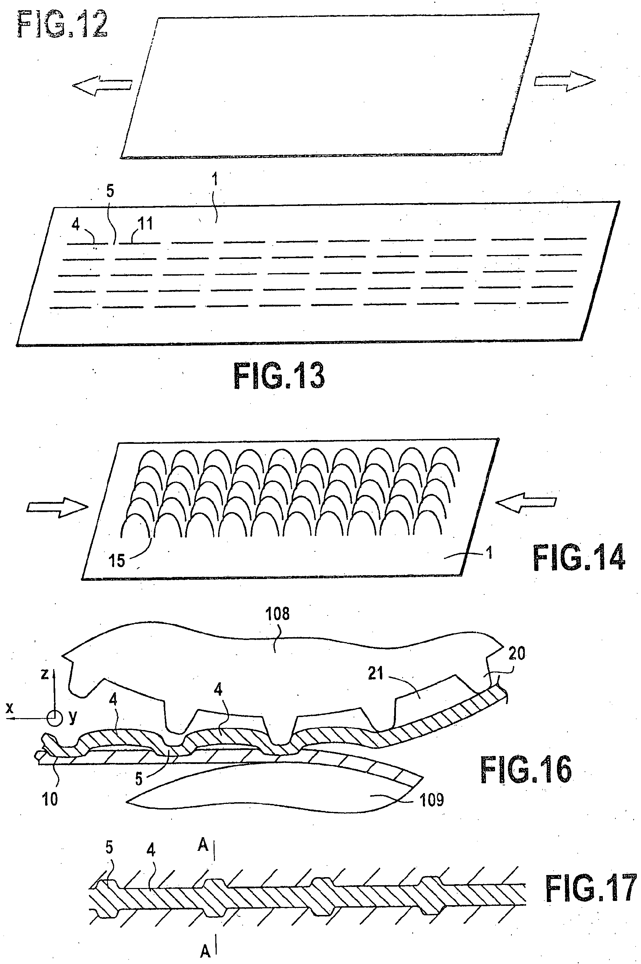

A la figure 1, l'élément à boucles comporte un film 1 en matière thermoplastique sur lequel ont été déposés des filaments 2 qui s'étendent parallèlement les uns aux autres dans une direction longitudinale (de droite à gauche aux figures). Pour une vue d'ensemble de cet élément, on peut notamment se reporter à la figure 13 (correspondant à l'état de la figure 1) et à la figure 14 (correspondant à l'état de la figure 2). Une fois les filaments positionnés sur le film 1, il est réalisé une préfixation le long de tronçons 5 de préfixation. Ainsi, le long de chaque filament s'étendent successivement des tronçons 4 non fixés et destinés à former une boucle et des tronçons 5 préfixés. Les tronçons 5 fixés sont ici sous la forme de segments de droite.

Une fois la préfixation des filaments sur le film thermoplastique effectuée, il est réalisé un traitement thermique qui a pour effet d'une part de rétracter le film 1 et d'autre part de ramollir simultanément les filaments dans les tronçons 5 de préfixation. Il en résulte une fixation très forte des filaments au film au niveau de tronçons de fixation 15 (ces tronçons 15 de fixation correspondent aux tronçon 5 de préfixation, mais n'en ont pas les mêmes dimensions, notamment longueur en raison de la rétraction liée au traitement thermique). En outre, il se forme des bulbes 6 au niveau des pieds de tronçons 4 libres, entre chaque tronçon 15 de fixation et le tronçon 4 libre suivant.

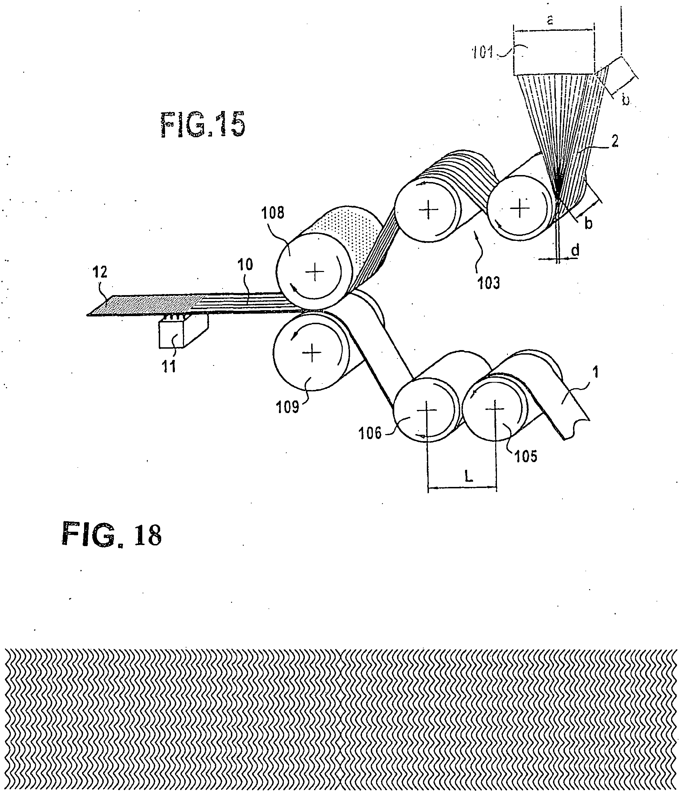

A la figure 15, il est représenté un dispositif pour la fabrication d'un élément à boucle.

La filière 101 permet d'extruder une multitude de filaments 2. Les trous d'extrusion des filaments sont disposés de façon à obtenir un faisceau de filaments de dimension a x b en sortie de filière. Ce faisceau passe à une dimension d x b, à l'arrivée au cabestan 103, avec d=diamètre des filaments. La disposition des trous dans la filière permet d'obtenir des filaments positionnés à intervalles réguliers, les uns à côté des autres à l'arrivée au cabestan 103. Le cabestan 103 permet également de refroidir les filaments. A ce niveau, les filaments ont un titre de 1 à 22 dtex, de préférence de 8 à 9 dtex.

Le film 1 arrive sur le cylindre 105. Son épaisseur est par exemple de 20 µm, de préférence de 15 à 25 µm. Elle peut également être comprise entre 10 et 100 µm. Le cylindre 105 tourne à une vitesse V, par exemple 100 m/min. Il est chauffé à une température en général égale ou supérieure au Point Vicat du film, par exemple 115°C. Un faible jeu est présent entre les deux cylindres 105 et 106, ce jeu est compris en général entre 10 et 100 µm, selon le film employé. Par exemple, pour un film 1 de 20 µm, l'entrefer sera de 20 µm.

Le cylindre 106 tourne à une vitesse V' supérieure à V dans le sens opposé. par exemple, V' = 2xV, soit 200 m/min. Le film 1 est alors étiré. Le cylindre 106 est refroidi entre 15 et 25°C, permettant au film de se figer dans son état d'étirage.

L'épaisseur du film, une fois étiré, passe, selon l'exemple, à une épaisseur égale à la moitié de l'épaisseur initiale, ici 10 µm. Le film est ensuite amené au module de positionnement et préfixation.

Les filaments 2 sont positionnés sur le film à l'aide d'un cylindre 108 gravé de pressage et d'un cylindre 109 lisse de support. Ces deux cylindres appliquent une pression sur le produit. Le premier objectif de cette étape est de mettre en contact les filaments et le film. Ces derniers sont mis en relation en des zones distinctes correspondant aux tronçon de préfixation. Pour que ce positionnement soit maintenu malgré le parcours du produit à travers la machine, il est nécessaire d'empêcher le filament et le film de bouger l'un par rapport à l'autre. L'étape de positionnement des filaments va donc nécessiter de comprimer les deux éléments l'un contre l'autre, de manière à créer des formes complémentaires. Cette opération peut-être assimilée à un pré ancrage mécanique des deux éléments. Cet ancrage est insuffisant pour créer une liaison dans le sens de la profondeur du film mais est suffisante pour maintenir les filaments sur le film dans le plan superficiel. Pour mettre en contact le film et les filaments dans des zones précises et leurs donner des formes complémentaires, on utilise deux cylindres 108 et 109. Des gravures sont effectuées dans le cylindre 108 de manière à former des zones 20 surélevées. Les deux cylindres sont mis en pression l'un contre l'autre comprimant ainsi le produit qui passe entre les deux au niveau des zones 20 surélevées pour former les tronçons 5 de préfixation. La pression est uniquement appliquée au niveau des zones 20 surélevées. En particulier, au niveau des zones 21 intermédiaires entre deux zones surélevées, les filaments et le film ne sont pas pressés l'un contre les autres. Il est ainsi former les tronçons 4 de boucles. Pour faciliter la déformation des produits, les deux cylindres 108, 109 sont chauffés. Les températures sont supérieures aux températures de déformation sous charges des polymères (Heat Deflection Temperature). Côté film, la température du cylindre doit être inférieure à la température de thermo rétraction du film, 100°C dans l'exemple.

Le pré positionnement et la préfixation des filaments sur le film s'effectuent de sorte que la liaison entre le filament et le film en matière thermo plastique reste superficielle. Ainsi, la liaison est réalisée de sorte qu'il y a certes contact mais pas interpénétration des molécules constituant respectivement le filament et le film. En particulier, on peut décoller les filaments du film sans grande difficulté. Cependant, les filaments sont déformés par rapport à leur forme initiale, notamment au niveau des tronçons 5 qui ont une section transversale plus oblongue allongée que la section transversale initiale (en général circulaire) des filaments.

Le complexe 10 formé par le film et les filaments à la sortie des cylindres 108, 109 est ensuite chauffé par un dispositif de chauffage 11. Il peut notamment s'agir d'un dispositif de chauffage par de l'air chaud, ou du plasma, ou des rayonnements tels que des infrarouges ou un laser, ou tout autre moyen d'apport d'énergie.

Il est préférable que le dispositif de chauffage soit disposé du côté du film opposé aux filaments (comme représenté à la figure 15). Ainsi, la chaleur attaque d'abord le film pour le thermo rétracter tandis que les filaments restent en partie protégés de la chaleur et ne font que se ramollir le temps du traitement thermique.

Cette étape permet de rétracter le film et de ramollir les filaments au niveau des tronçons de préfixation. On obtient alors un produit avec des boucles, intimement liées au film et renforcées à leur base.

Par exemple, si la valeur de rétraction est de 2, l'épaisseur du fond du produit redevient 20 µm. Si la vitesse d'entrée de cette étape est de 200 m/min, le produit sortira alors à 100 m/min.

L'aire de section transversale (perpendiculairement au filament) des filaments au niveau des tronçons de boucle 4, hors bulbes 6, est sensiblement égale à l'aire de la section transversale du filament de départ. Elle est sensiblement constante d'un bulbe à l'autre. Les bulbes 6 s'étendent chacun à partir de la section sensiblement constante des boucles 4 jusqu'au tronçon 15 de fixation. Les deux bulbes 6 voisins sont séparés par un tronçon 15 de fixation. Le tronçon 15, entre les deux bulbes, a une aire de section transversale sensiblement constante. La section transversale du tronçon 15 entre les deux bulbes 6 est de forme aplatie par rapport à celle des sections des tronçons 4 libres formant boucle. L'aire de la section transversale du tronçon 15 qui s'étend entre les deux bulbes 6 est plus grande que l'aire de la section des tronçons 4, hors bulbes, et notamment de la section du filament au niveau du sommet (à mi distance entre les deux bulbes) de chaque boucle 4. Elle est en outre de forme aplatie notamment ovale ou légèrement rectangulaire.

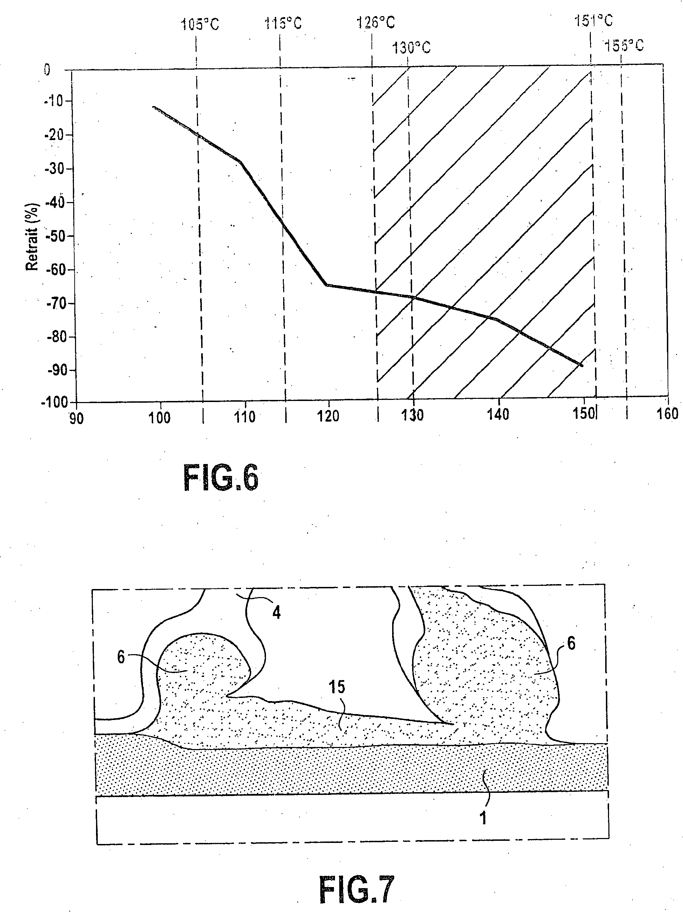

Ainsi, en prévoyant un traitement thermique dont la température de traitement est supérieure au Point Vicat des matériaux du film, et de préférence également des filaments, on s'assure, avec la rétraction thermique concomitante du film, une excellente fixation des filaments. En outre, dans le cas où l'on choisit une température supérieure à la température Vicat des filaments, le ramollissement des filaments entraîne une déformation des filaments et la formation notamment des deux bulbes 6 au niveau des pieds des boucles qui ont pour effet d'améliorer la résistance à la traction et à la déchirure de ces boucles qui ainsi résistent mieux dans leur fonction de boucle dans un autoagrippant à crochets et boucles. Comme représenté à la figure 6, la plage de travail préféré est comprise entre le point Vicat le plus élevé parmi le Point Vicat des filaments et du film et le point de fusion le plus bas des points de fusion du film et des filaments. La figure 6 a été réalisée à titre d'exemple sur la base d'un film en PEBD et en PP et de filaments en PP.

A la figure 4, il est représenté un exemple de mode de réalisation possible concernant les matériaux choisis. Les filaments 2' sont en polypropylène (PP) tandis que le film 1' est constitué d'un stratifié à trois couches comportant deux couches extérieures de polypropylène (PP) et d'une couche intérieure en polyéthylène basse densité (PEBD). Ainsi, les couches extérieures favorisent la liaison intime avec les filaments tandis que le polyéthylène basse densité intérieur va réaliser la thermo rétraction.

Un autre mode de réalisation possible consiste à réaliser le film 1" en polyéthylène basse densité (PEBD) dans sa totalité (voir la figure 5) tandis que les filaments 2" sont constitués chacun d'une gaine extérieure en polyéthylène basse densité (PEBD) pour favoriser la liaison intime avec le film, et d'une âme en polypropylène (PP) pour leur propriété mécanique.

En outre de préférence, les filaments peuvent être réalisés en POY, en MOY ou en LOY. Ceci à l'avantage d'augmenter la résistance au pelage ou traction lorsque l'élément femelle est utilisé avec des crochets. En outre, ceci améliore la douceur au contact de la peau notamment des bébés.

Les filaments de type LOY sont des filaments non étirés ou très faiblement étirés. En filature, ces filaments sont bobinés sous la filière, à basse vitesse, de l'ordre de 500 à 600 m/mn, sans passage par un procédé d'étirage. Les filaments obtenus ont ainsi une grande capacité d'allongement, en général supérieure à 195% de la longueur initiale (longueur initiale de 100% + allongement de 95%= 195% de la longueur initiale). Les filaments PP utilisés dans les modes de réalisation fournis à titre d'exemple de la présente invention peuvent avoir un allongement de 500% de leur longueur initiale (la longueur du filament après allongement étant alors de 100 + 400= 500% de la longueur initiale).

Les filaments de type POY (Partially Oriented Yarn) sont des filaments faiblement étirés, de l'ordre d'un facteur 2. Le filament obtenu a alors un allongement moindre que le LOY mais toujours supérieur à 160% (longueur initiale de 100% + allongement de 60%= 160% de la longueur initiale)

Enfin ils existent également des filaments intermédiaire, appelés MOY, dont l'allongement peut atteindre 180%.

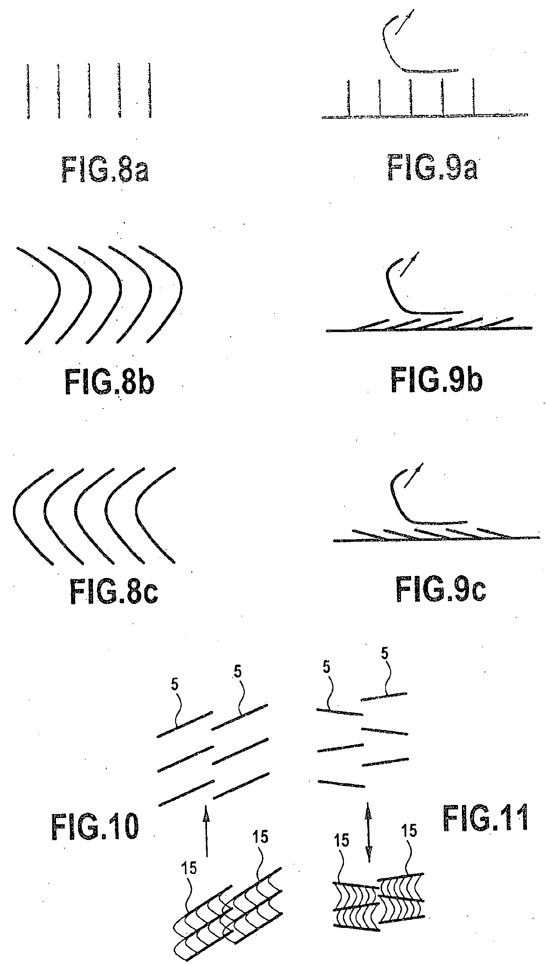

Lorsque l'on réalise la préfixation des tronçons de fixation, on peut faire en sorte que les tronçons de préfixation soient décalés les uns par rapport aux autres d'un filament à l'autre. On peut ainsi se reporter aux figures 10 ou 11 dans laquelle il est représenté des motifs formés par les tronçons 5 de préfixation après préfixation et les motifs formés par les tronçon 15 de fixation après traitement thermique. En disposant ainsi les tronçons de préfixation décalés transversalement aux filaments, on s'assure, comme représenté, que les boucles, après le traitement thermique, seront orientées vers la gauche ou la droite, en fonction du motif choisi. Lorsque l'on.veut que la boucle qui s'étend entre deux lignes de fixation consécutives (les lignes sont en fait des successions de tronçons 5 ou 15 et ces successions sont représentées schématiquement sous la forme de lignes, alors même qu'il ne s'agit pas à proprement parler de lignes puisque qu'il y a discontinuité entre deux tronçons de fixation successif dans une ligne, les filaments étant à distance les uns des autres) soit orientées vers la gauche, on réalise la ligne croissante de gauche à droite (voir la figure 10 pour un sens de défilement vers le haut de la figure).

Si l'on souhaite que les boucles soient orientées vers la droite, on réalise à l'inverse de la figure 10, les lignes décroissantes de gauche à droite.

On peut également réaliser un motif quelconque comportant, comme on le souhaite, des boucles orientées à gauche et à droite, comme l'exemple à la figure 11 qui est particulièrement avantageux en terme de résistance au pelage.

Ainsi, comme représenté en figure 8c et 9c, les boucles sont orientées dans la direction opposée à la direction d'ouverture. Ceci permet d'obtenir des forces de pelage supérieures à celles de la configuration des figures 8a et 9a et très supérieures à celles de la configuration des figures 8b et 9b. Pour obtenir des performances très élevées sur une bande confort de couche culotte, la disposition représentée à la figure 18 est particulièrement avantageuse. Les boucles y sont orientées vers la gauche sur la moitié de la bande confort devant permettre l'ouverture à droite et inversement pour la partie gauche. Cette disposition des boucles pourrait certes également être obtenue par l'utilisation d'un rouleau d'orientation pendant et après la thermorétraction. Cette dernière méthode n'est cependant pas aussi précise que d'effectuer l'orientation par le motif lui même comme suivant l'aspect de l'invention mentionné plus haut.

On peut bien évidemment dans une Bande Confort réaliser des boucles vers la gauche et suivant le principe inverse réaliser d'autres boucles vers la droite. On peut également réaliser des boucles qui dans un filament donné sont alternativement orientées à gauche et à droite comme représenté à la figure 11.

Suivant l'invention, le film a une épaisseur qui est sensiblement constante que l'on se trouve au niveau des tronçons de fixation ou des tronçons de boucles, en particulier, l'épaisseur dans la zone des tronçons de fixation est inférieure d'au plus 25% à l'épaisseur du film à l'extérieur de ces zones de fixation et notamment inférieure d'au plus 20%, encore plus préférablement inférieure d'au plus 10%.

De préférence le film est thermo rétractable. Les films thermo rétractables sont généralement utilisés dans le domaine du packaging. L'application principale est la mise sous film plastique d'un lot de produits. Une fois ensaché, le lot passe dans un four, le film se rétracte et se tend sur les produits. Cela crée un conditionnement efficace.

Ces films sont généralement bi orientés, de façon à être thermo rétractables dans toutes les directions. Pour obtenir de tels films, le film initial, généralement à base d'éthylène, est étiré dans les deux directions.

Le film 1 obtenu, voir à la figure 1, est thermo rétractable. Il suffit ensuite de la chauffer à une température adaptée pour qu'il se rétracte. Le procédé pour obtenir cette bi orientation est l'augmentation progressive des vitesses des cylindres de tirage en MD et l'utilisation de galet pour étirer le film latéralement.

Contrairement aux films thermo rétractables classiques, le film employé est de préférence thermo rétractable uniquement en MD, ceci pour éviter le rebut en largeur et également pour maîtriser parfaitement la densité de filaments. Le film va donc être étiré dans une seule direction. Pour obtenir un film thermo rétractable mono orienté, il faut l'étirer uniquement dans une direction.

Le produit obtenu est bien thermo rétractable en MD mais a tendance à gonfler en largeur sous l'effet de la température.

A la figure 6, il est représenté un graphe donnant le retrait du film en fonction de la température du traitement thermique. Ce graphe a été réalisé pour un film à trois couches de PP, PEBD et PP, tandis que les filaments sont en PP. Le point de fusion du PEBD est 105°C et le point Vicat A du PP est 115°C, tandis que le point Vicat A de la matière PP des filaments est de 126°C.

On choisit ainsi une température de 130° C pour le traitement thermique, supérieure aux points Vicat A ci dessus des matières du film et des filaments et inférieure à la température de fusion du PP du film. (Point de fusion du PP est de 151°C) et des filaments (155°C).

Dans la présente invention, on entend par thermo rétractable un élément, notamment un film, qui se rétracte d'au moins 10% (de préférence d'au moins 20%) lorsqu'il est porté à une température inférieure de 10° à son point de fusion.

## Revendications

1. Installation destinée à fabriquer un élément femelle à boucle, qui comporte :
- des moyens pour amener un faisceau de filaments (2 ; 2' ; 2") et un film (1 ; 1' ; 1") en matière thermoplastique en contact mutuel dans un interstice compris entre une surface de pressage, notamment la surface d'un cylindre (108) de pressage, et une surface de support, notamment la surface d'un cylindre (109) de support,
- la surface de pressage étant gravée de sorte qu'il y est formé dés zones. (20) de surélévation destinées à presser de manière sélective des tronçons des filaments contre le film tandis qu'au niveau des parties (21) non surélevées de la surface de pressage des tronçons des filaments ne sont pas pressés contre le film ;
**caractérisée en ce que**
- il est prévu un poste (11) de chauffage pour chauffer l'ensemble film-filaments sortant de l'interstice, de sorte qu'en sortie de l'installation, des filaments comportent une succession de tronçons (15) de fixation et une succession de tronçons (4) de boucles, les filaments étant fixés au film le long des tronçons (15) de fixation tandis que les tronçons (4) de boucles sont à distance du film pour former les boucles, et l'aire d'une section transversale d'un tronçon (4) de boucle d'un filament est inférieure à l'aire d'une section transversale d'un tronçon (15) de fixation dudit filament.

2. Installation suivant la revendication 1, **caractérisée en ce que** le faisceau de filaments forme un rideau de filaments.

3. Installation suivant l'une des revendications précédentes, **caractérisée en ce que** les filaments s'étendent parallèlement les uns aux autres.

4. Installation suivant l'une des revendications précédentes, **caractérisée en ce que** la surface de pressage est la surface d'un cylindre (108) de pressage.

5. Installation suivant l'une des revendications précédentes, **caractérisée en ce que** la surface de support est la surface d'un cylindre (109) de support.

6. Installation suivant l'une des revendications précédentes, **caractérisée en ce que** le dispositif de chauffage est disposé du côté du film opposé aux filaments.

7. Installation suivant l'une des revendications précédentes, **caractérisée en ce que** pour faciliter la déformation des produits les deux cylindres de pressage et de support sont chauffés, les températures étant supérieures aux températures de déformation sous charges des polymères (Heat Deflection Température), la température du cylindre côté film restant inférieure à la température de thermo rétraction du film.

8. Installation suivant l'une des revendications précédentes, **caractérisée en ce que** avant de parvenir à l'interstice, le film passe entre un cylindre (105) tournant à une vitesse V et un cylindre (106) tournant à une vitesse V' supérieure à V et de sens opposé, de sorte que le film soit étiré.

9. Installation suivant la revendication 8, **caractérisée en ce que** il est présent un faible jeu entre les deux cylindres (105, 106).

10. Installation suivant la revendication 8, ou 9, **caractérisée en ce que** le cylindre (105) est chauffé à une température en général égale ou supérieure au Point Vicat du film et le cylindre (106) est refroidi entre 15 et 25°C, permettant au film de se figer dans son état d'étirage.

11. Installation suivant l'une des revendications 1 à 10 **caractérisée en ce que** le film, une fois étiré, est amené dans l'interstice.

## Patentansprüche

1. Eine Anlage zur Herstellung eines weiblichen Schlaufenelements, umfassend:
Mittel zum Einführen eines Filamentbündels (2; 2'; 2") und einer Folie (1; 1', 1") aus thermoplastischem Material, die in gegenseitigem Kontakt stehen, in einen Spalt zwischen einer Pressoberfläche, mit der Oberfläche eines Presszylinders (108), und einer Stützoberfläche, mit der Oberfläche eines Stützzylinders (109),
wobei die Pressoberfläche derart geprägt ist, dass Erhebungen (20) auf ihr ausgebildet sind, um selektiv Filamentabschnitte gegen die Folie zu pressen, während in den nicht erhabenen Bereichen (21) die Pressoberfläche die Filamente nicht gegen die Folie drückt;
**dadurch gekennzeichnet, dass**
eine Heizvorrichtung (11) vorgesehen ist, um die den Spalt verlassende Folien-Filament-Anordnung zu erhitzen, sodass die Filamente beim Verlassen der Anlage eine Reihe von Befestigungsabschnitten (15) und eine Reihe von Schlaufenabschnitten (4) aufweisen,
wobei die Filamente auf der Folie entlang der Befestigungsabschnitte (15) haften, während die Schlaufenabschnitte (4) sich in einem Abstand zu der Folie befinden, um die Schlaufen zu bilden, und
wobei die Querschnittsfläche eines Schlaufenabschnitts (4) eines Filaments kleiner ist als die Querschnittsfläche eines Befestigungsabschnitts (15) des besagten Filaments.

2. Eine Anlage gemäß Anspruch 1, **dadurch gekennzeichnet, dass** das Filamentbündel einen Filamentvorhang bildet.

3. Eine Anlage gemäß einem der vorigen Ansprüche, **dadurch gekennzeichnet, dass** die Filamente parallel zueinander angeordnet sind.

4. Eine Anlage gemäß einem der vorigen Ansprüche, **dadurch gekennzeichnet, dass** die Pressoberfläche die Oberfläche eines Presszylinders (108) ist.

5. Eine Anlage gemäß einem der vorigen Ansprüche, **dadurch gekennzeichnet, dass** die Stützoberfläche die Oberfläche eines Stützzylinders (109) ist.

6. Eine Anlage gemäß einem der vorigen Ansprüche, **dadurch gekennzeichnet, dass** die Heizvorrichtung an der den Filamenten gegenüberliegenden Seite der Folie angeordnet ist.

7. Eine Anlage gemäß einem der vorigen Ansprüche, **dadurch gekennzeichnet, dass** zur leichteren Verformung der Produkte sowohl der Presszylinder als auch der Stützzylinder erhitzt werden,
wobei die Temperatur höher ist als die Wärmeformbeständigkeitstemperatur (Heat Deflection Temperature),
wobei die Temperatur des folienseitigen Zylinders niedriger bleibt als die thermische Schrumpfungstemperatur der Folie.

8. Eine Anlage gemäß einem der vorigen Ansprüche, **dadurch gekennzeichnet, dass** die Folie, bevor sie den Spalt erreicht, zwischen einem Zylinder (105), der mit einer Geschwindigkeit V rotiert, und einem Zylinder (106), der mit einer Geschwindigkeit V' in die entgegengesetzte Richtung rotiert, die größer ist als V, hindurch tritt, so dass sie gedehnt wird.

9. Eine Anlage gemäß Anspruch 8, **dadurch gekennzeichnet, dass** ein geringer Spalt zwischen den beiden Zylindern (105, 106) besteht.

10. Eine Anlage gemäß Anspruch 8 oder 9, **dadurch gekennzeichnet, dass** der Zylinder (105) auf eine Temperatur erhitzt wird, die im Allgemeinen gleich oder höher als der Vicat-Erweichungspunkt ("Point Vicat") der Folie ist, und dass der Zylinder (106) auf eine Temperatur zwischen 15°C und 25°C gekühlt wird, wodurch die Folie in ihrem gestreckten Zustand erstarrt.

11. Eine Anlage nach einem der Ansprüche 1 bis 10, **dadurch gekennzeichnet, dass** die Folie, ein Mal gedehnt, in den Spalt eingeführt wird.

## Claims

1. Installation intended for producing a female loop portion which comprises :
- means for bringing a curtain of filaments (2 ; 2' ; 2") and a film (1 ; 1'; 1") made of thermoplastic material into mutual contact in a gap between a pressing surface, in particular the surface of a pressing cylinder (108), and a supporting surface, in particular the surface of a supporting cylinder (109),
- the pressing surface being engraved so as to form thereon raised zones (20) intended to press selectively sections of the filaments against the film, while in the region of the non-raised portions (21) of the pressing surface, sections of the filaments are not pressed against the film ; **characterized in that**
- there is provided a heating device (11) to heat the film-filaments assembly leaving the gap, so that, at the exit of the installation, filaments comprise a series of fixating portions (15) and a series of loop portions (4), the filaments being fixated to the film along the fixating portions (15) while the loop portions (4) are distant from the film so as to form loops, and the area of a cross section of a loop portion (4) of a filament is less than an area of a cross section of a fixating section (15) of said filament.

2. Installation according to Claim 1, **characterized in that** the bundle of filaments form a curtain of filaments.

3. Installation according to one of the preceding claims, **characterized in that** the filaments extend parallel to one another.

4. Installation according to one of the previous claims, **characterized in that** the pressing surface is the surface of a pressing cylinder (108).

5. Installation according to one of the previous claims, **characterized in that** the support surface is the surface of a support cylinder (109).

6. Installation according to one of the preceding claims, **characterized in that** the heating device is disposed on the side of the film which is opposite to the filaments.

7. Installation according to one of the preceding claims, **characterized in that** to facilitate the deformation of the products, the two pressing and support cylinders are heated, the temperatures being higher than the temperatures at which the polymers deform under load (Heat Deflection Temperature), the temperature of the cylinder on the film side staying below the temperatures of thermo-retracting of the film.

8. Installation according to one of the previous claims, **characterized in that** before arriving at the gap, the film passes between a cylinder (105) rotating at a speed V and a cylinder (106) rotating at a speed V' higher than V and of opposite direction, so that the film is stretched.

9. Installation according to claim 8, **characterized in that** a small gap is present between the two cylinders (105, 106).

10. Installation according to claim 8 or 9, **characterized in that** the cylinder (105) is heated at a temperature generally equal or higher than the Vicat temperature of the film and the cylinder (106) is cooled between 15 and 25°C, allowing the film to tighten in its stretched state.

11. Installation according to one of claims 1 to 10, **characterized in that** the film, once stretched, is brought in the gap.
